# EUROPEAN PATENT APPLICATION

(11) **EP 1 894 521 A1**
(43) Date of publication of application: **05.03.2008**
(21) Application number: 07253351.6
(22) Date of filing: 24.08.2007
(51) Int. Cl.: A61B 5/024

(54) **Heartbeat information acquiring apparatus**

(30) Priority: 30.08.2006 JP 2006233172
(71) Applicant: Seiko Instruments Inc., Chiba-shi, Chiba (JP)
(72) Inventor: Fujiwara, Toshiyuki, c/o Seiko Instruments Inc, Chiba-shi, Chiba (JP)
(74) Representative: Cloughley, Peter Andrew

(57) **Abstract**

A heartbeat information acquiring apparatus comprises an electrode unit comprising a pair of chest electrodes (11A,11B) attached to a main unit belt (1), a heartbeat detecting unit for detecting a heartbeat signal based on a cardio-potential produced between the pair of chest electrodes, an arithmetic and control unit (13), and a reporting unit (16,17). The arithmetic and control unit (13) comprises a heartbeat rate computing means for computing a heart rate by processing the heartbeat signal, control means for controlling the reporting unit (16,17) to allow the reporting unit to report heartbeat information to the user, and the arithmetic and control unit (13) is attached to the main unit belt (1) by being accommodated in the main unit case.

## Description

The present invention relates to a heartbeat information acquiring apparatus that processes a user's cardio-potential produced between a pair of electrodes attached on a fitting belt to detect an electrocardiograph signal, processes the electrocardiograph signal to calculate a heart rate, and allows the user to acquire heartbeat information based on the heart rate.

A heart rate meter commonly comprises: a detection unit for detecting a heartbeat signal by processing a cardio-potential produced between a pair of chest electrodes attached on a fitting belt; a heartbeat signal transmitter having a wireless transmission unit for transmitting the heartbeat signal; and a heartbeat signal receiver having a wireless receiver unit for receiving the heartbeat signal, an arithmetic circuit for processing the heartbeat signal to calculate a heart rate, and a display unit for displaying the heart rate based on the heart rate. The heartbeat signal transmitter is fitted on the chest part of the user with the chest belt, and the heartbeat signal receiver is a wristwatch or a wristwatch-type receiver fitted on a wrist of the user.

The above-described heart rate meter, which transmits/receives a heartbeat signal wirelessly, can carry out highly reliable, accurate heartbeat measurement under an environment in which no radio interference occurs. The above-described heart rate meter, however, has a problem that radio interference occurs and accurate heartbeat measurement is impossible in such a case that a plurality of users are simultaneously using the heart rate meters that transmit/receive heartbeat signals wirelessly in close proximity to one another. The above-described heart rate meter that transmits/receives a heartbeat signal wirelessly often cannot measure an accurate heart rate in the case that, for example, the user conducts heartbeat measurement while using a training machine, since the heart rate meter is affected by external noise due to the electromagnetic wave generated by the training machine. Thus, various types of heart rate meters that are not affected by external noise have been proposed.

Japanese Patent No. 3,568,954 discloses a method of transmitting a heartbeat signal without being interfered by radio interference. According to this method, a heartbeat signal transmitter fitted on the user's chest by a chest belt detects an electric signal generated by a pulsation of the heart, conducts filter-amplification, wave-detection, and wave-shaping, and transmits an electrocardiograph signal with a burst wave. The heartbeat signal transmitter comprises a pair of chest electrodes attached on the chest belt. This burst wave electrocardiograph signal is received by a reception antenna that is built in a wristwatch, which is the heartbeat signal receiver. Based on the reception signal, a heart rate is calculated, and the heart rate is displayed on a display unit of the wristwatch. Thereby, it intends to prevent radio interference.

The conventional configuration that detects a heartbeat signal by a pair of chest electrodes fitted on the chest belt and transmits it by a burst wave to the heartbeat signal receiver, however, has the following problem. In the case that it is adversely affected by external noise, for example, in the case of using a training machine, accurate burst wave cannot be detected because of the adverse effects of the noise generated by the training machine even when a heartbeat signal is transmitted, so even when the wristwatch that is the heartbeat signal receiver processes the received burst wave heartbeat signal, an accurate heart rate cannot be obtained. Moreover, it has been difficult for the conventional configuration in which the burst signal is transmitted to measure an accurate heart rate when similar devices are used in close proximity to one another, since the apparatus receives a plurality of burst signals.

In Domestic Re-publication of PCT patent Application No. 96/029005, the apparatus comprises a transmitter and a receiver. The transmitter comprises: a detection circuit for detecting an electrocardiograph signal obtained from a pair of electrodes fitted near the heart in a human body and generating a detection signal; an interval data producing circuit for counting the generation interval of the detection signal with a certain periodic signal and generating interval data; a serial data producing circuit for converting the interval data into a serial signal and generating a serial data signal; a transmitter having a transmission circuit for modulating the serial data signal and transmitting it as a wireless signal; and a battery as a power supply. The receiver comprises: a reception circuit for receiving the wireless signal from the transmitter, demodulating the signal, and generating a reception signal; a data decode circuit for decoding the reception signal and generating electrocardiograph interval data, an arithmetic circuit for calculating a heart rate from the electrocardiograph interval data; and a display unit for displaying the heart rate.

In this conventional heart rate meter, even when the receiver receives noise or irrelevant signals, the data decode circuit cannot properly decode the data since they are not proper data, so the previous data at the time of proper reception is displayed. Therefore, the conventional heart rate meter does not display erroneous heart rates. Nevertheless, a problem with this conventional heart rate meter is that both the transmitter and the receiver are complicated, and accordingly, the manufacturing cost is high. Moreover, another problem is that, when the heart rate meter is continuously affected by external noise, as in the case that the user is using a training machine, the user cannot provide real-time heart rates. The reason is that the above-described receiver is not the one that prevents the reception of noise radio wave.

A problem that the invention is intended to solve is to provide a low-cost apparatus that allows the user to acquire accurate heartbeat information at all times without being adversely affected by radio interference or noise under any environment

In order to resolve the foregoing problem, an analysis was conducted on the behavior of the users of the conventional heart rate meter that comprises a transmitter attached on the fitting belt and a wristwatch-type receiver. As a result, it has been found that the user looks at the display unit of the wristwatch-type receiver while running in the case where the user himsel f/herself consciously confirms the heart rate, and that the confirmation of the heart rate is not frequent. In short, a heart rate is a guideline for exercise intensity. The user considers it as a guideline for deciding to slow down the pace since the exercise intensity is high when the heart rate is too high, or to speed up the pace since the exercise intensity is low when the heart rate is low. Also, there are cases in which the user stops or slows down the essential running action when he/she performs a special viewing action of looking at the display unit of the wristwatch-type receiver. According to the results of the analysis of the user behavior, the user demands heartbeat information indicating that the heart rate is whether or not within a preset range by the user, rather than a real-time heart rate while doing exercise.

An apparatus that solves the foregoing problem is an apparatus comprising: an electrode unit comprising a main unit case attached on a fitting belt and a pair of chest electrodes; a heartbeat detecting unit for detecting a heartbeat signal based on a cardio-potential produced between the pair of chest electrodes; an arithmetic and control unit, and a reporting unit, the apparatus to be fitted to a chest part of a user with the fitting belt. The arithmetic and control unit comprises heartbeat rate computing means for computing a heart rate by processing the heartbeat signal, control means for comparing the heart rate and a target value and controlling the reporting unit to allow the reporting unit to report heartbeat information to the user, and the arithmetic and control unit is accommodated in the main unit case. The heartbeat information is all the kinds of, or one or two kinds of, information among information to the effect that the heart rate is within a set range, information to the effect that the heart rate has exceeded an upper limit target value, and information to the effect that the heart rate has fallen below a lower limit target value.

The user of the heartbeat information acquiring apparatus according to the invention has been able to acquire accurate heartbeat information at all times without being adversely affected by radio interference or noise under any environment. Moreover, the heartbeat information acquiring apparatus according to the invention does not require a transmitter circuit and a receiver circuit, and the circuit configuration is simpler than conventional apparatus, making it possible to reduce manufacturing costs. Furthermore, since the heartbeat information is notified by sound or vibration, the user does not need to take a special viewing action of looking at the display, and does not need to interrupt or slow down the essential running or training action.

Embodiments of the present invention will now be described by way of further example only and with reference to the accompanying drawings, in which:
Fig 1 is a perspective view of the heartbeat information acquiring apparatus of example 1.
Fig. 2 is a partially-enlarged plan view of the principal portion of the heartbeat information acquiring apparatus of example 1.
Fig. 3 is a block circuit diagram of the heartbeat information acquiring apparatus of example 1.
Fig. 4 is a view for illustrating the state in which the heartbeat information acquiring apparatus of example 1 is fitted to the chest part of a user.

A heartbeat information acquiring apparatus according to the invention comprises: an electrode unit having a main unit case attached on a fitting belt and comprising a pair of chest electrodes; a heartbeat detecting unit for detecting a heartbeat signal based on a cardio-potential produced between the pair of chest electrodes; an arithmetic and control unit, and a reporting unit, and it is an apparatus to be fitted to a chest part of a user with the fitting belt. The arithmetic and control unit comprises heartbeat rate computing means for computing a heart rate by processing the heartbeat signal, control means for comparing the heart rate and a target value and controlling the reporting unit to allow the reporting unit to report heartbeat information to the user, and the arithmetic and control unit is accommodated in the main unit case. The heartbeat information is all the kinds of, or one or two kinds of, information among information to the effect that the heart rate is within a set range, information to the effect that the heart rate has exceeded a upper limit target value, and information to the effect that the heart rate has fallen below a lower limit target value. The reporting unit is a vibrating motor and is attached to the fitting belt at such a position as to transmit vibration to the user's sternum.

### Embodiment 1

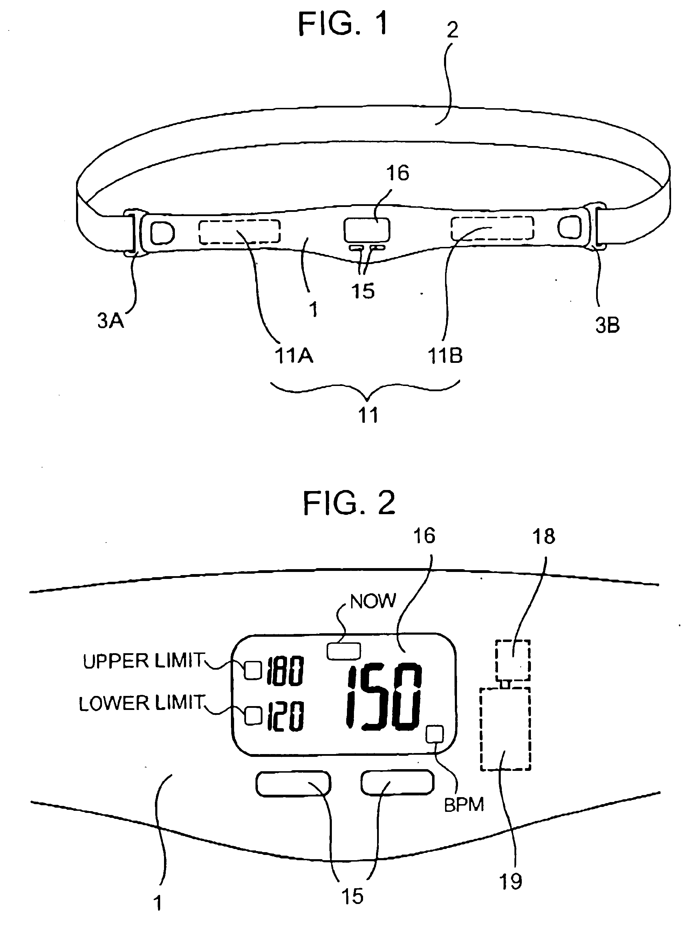

As illustrated in the perspective view shown in Fig. 1 and the partially-enlarged plan view of its primary portion shown in Fig. 2, a heartbeat information acquiring apparatus according to example 1 of the invention has a fitting belt in which a main unit belt 1 and both respective ends of a back belt 2 are fastened by fastening devices 3A and 3B. A main unit case, which is not shown in the figure, is mounted at the central portion of the main unit belt 1. An electrode unit 11 comprising a pair of chest electrodes 11A and 11B is buried in the main unit belt 1, near the left and right ends thereof.

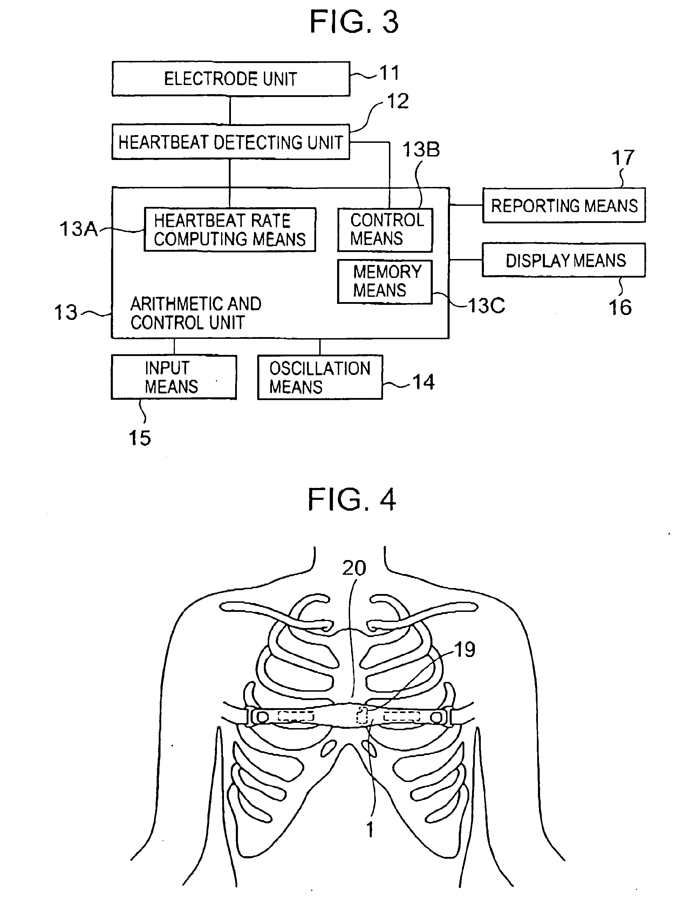

As illustrated in the block circuit diagram of Fig. 3, the heartbeat information acquiring apparatus according to example 1 of the invention comprises a heartbeat detecting unit 12 for detecting a heartbeat signal based on a cardio-potential produced between the pair of chest electrodes 11A and 11B, an arithmetic and control unit 13, and a reporting unit. The reporting unit comprises display means 16 and reporting means 17.

The arithmetic and control unit 13 comprises a heartbeat rate computing means 13A for computing a heart rate by processing the heartbeat signal, control means 13B for comparing the heart rate and a target value and controlling the display means 16 and reporting means 17 which serve as the reporting unit to report heartbeat information to the user, and memory means 13C in which programs and data are stored.

Oscillation means 14 is means for supplying a clock pulse to the arithmetic and control unit 13, and input means 15 is means such as an input button for allowing the user to set a target value in the arithmetic and control unit 13. The target value to be set includes a upper limit target value and a lower limit target value of the heart rate. If necessary, one of the upper limit target value or the lower limit target value of the heart rate may be set as the target value.

The display means 16 that makes up the reporting unit has a liquid crystal display panel mounted on the main unit case 1 and displays each of the current heart rate, the upper limit value of the heart rate, and the lower limit value of the heart rate. The display means 16 configures a part of the target value setting function when the input means 15 is operated.

The heartbeat information that the reporting unit 16, 17 reports is all the kinds of, or one or two kinds of, information among the current heart rate information (current information), the information to the effect that the heart rate has exceeded the upper limit value of the target (upper limit information), and the information to the effect that the heart rate has fallen below the lower limit value of the target (lower limit information). Herein, the heartbeat information in example 1 is assumed to be three kinds, the current information, the upper limit information, and the lower limit information.

The reporting means 17 that makes up the reporting unit may be voice reporting means, electronic sound reporting means, vibrating reporting means, and the like. In the case that the reporting means 17 is voice reporting means, heartbeat information with the content corresponding to the current information, the upper limit information, or the lower limit information is vocally reported by synthesized voice.

In the case that the reporting means 17 is electronic sound reporting means, heartbeat information is reported by electronic sound with a tone or a number of times of repetition that corresponds to the current information, the upper limit information, or the lower limit information.

Moreover, in the case that the reporting means 17 is vibrating reporting means such as a vibrating motor 19, heartbeat information is reported by vibration with a number of times of repetition that corresponds to the current information, the upper limit information, or the lower limit information. The vibrating motor 19, which serves as the reporting means, is attached to the main unit belt 1 at such a position as to transmit vibration to the user's sternum 20. Thus, the user can acquire heartbeat information very accurately because he/she receives the vibration that the vibrating motor 19 generates by his/her sternum 20. It should be noted that, as shown in Fig. 2, the vibrating motor 19 comprises a weight 18 for obtaining high vibration. It should be noted that the reporting means 17 may comprise not only one of voice reporting means, electronic sound reporting means, and vibrating reporting means, but also a plurality of means of combinations of these means.

The aforegoing description has been given by way of example only and it will be appreciated by a person skilled in the art that modifications can be made without departing from the scope of the present invention.

## Claims

1. A heartbeat information acquiring apparatus comprising an electrode unit comprising a pair of chest electrodes, a heartbeat detecting unit for detecting a heartbeat signal based on a cardio-potential produced between the pair of chest electrodes, an arithmetic and control unit, and a reporting unit, the heartbeat information acquiring apparatus to be fitted to a chest part of a user with a fitting belt, **characterized in that**:
the arithmetic and control unit comprises heartbeat rate computing means for computing a heart rate by processing the heartbeat signal, control means for comparing the heart rate and a target value and controlling the reporting unit to allow the reporting unit to report heartbeat information to the user.

2. The heartbeat information acquiring apparatus as set forth in claim 1, wherein the heartbeat information is all the kinds of, or one or two kinds of, information among information to the effect that the heart rate is within a set range, information to the effect that the heart rate has exceeded a upper limit target value, and information to the effect that the heart rate has fallen below a lower limit target value.

3. The heartbeat information acquiring apparatus as set forth in claim 1 or claim 2, wherein the reporting unit comprises at least one of voice reporting means, electronic sound reportingmeans, and vibrating reporting means.

4. The heartbeat information acquiring apparatus as set forth in claim 3, wherein the vibrating reporting means is a vibrating motor and is attached to the fitting belt at such a position as to transmit vibration to the user's sternum.

5. The heartbeat information acquiring apparatus as set forth in claim 4, wherein the vibrating motor comprises a weight for obtaining a high vibration.
